(19) Europäisches Patentamt
European Patent Office
Office européen des brevets

(11) Veröffentlichungsnummer: **0 008 084**
**B1**

(12) **EUROPÄISCHE PATENTSCHRIFT**

(45) Veröffentlichungstag der Patentschrift: **20.01.82**

(51) Int. Cl.³: **C 07 D 498/10,** C 08 K 5/35 // (C07D498/10, 263/00, 221/00)

(21) Anmeldenummer: **79102779.0**

(22) Anmeldetag: **02.08.79**

(54) **Neue Harnstoffderivate, ihre Herstellung und ihre Verwendung als Lichtschutzmittel für Polymere.**

(30) Priorität: **05.08.78 DE 2834455**

(43) Veröffentlichungstag der Anmeldung: **20.02.80 Patentblatt 80/4**

(45) Bekanntmachung des Hinweises auf die Patenterteilung: **20.01.82 Patentblatt 82/3**

(84) Benannte Vertragsstaaten: **CH DE FR GB IT NL SE**

(56) Entgegenhaltungen:

**DE-A-1 769 646**
**DE-A-1 770 689**
**DE-A-2 227 689**
**DE-A-2 500 313**
**DE-A-2 500 314**
**DE-A-2 606 026**
**DE-A-2 642 461**

(73) Patentinhaber: **HOECHST AKTIENGESELLSCHAFT, Postfach 80 03 20, D-6230 Frankfurt/Main 80 (DE)**

(72) Erfinder: **Wiezer, Hartmut, Dr., Hans-Fischer-Strasse 6, D-8906 Gersthofen (DE)**
Erfinder: **Mayer, Norbert, Dr., Ziegelgrundweg 17, D-8901 Gablingen (DE)**
Erfinder: **Knorr, Harald, Dr., Hans-Fischer-Strasse 6, D-8906 Gersthofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 008 084 B1

ACTORUM AG.

# Neue Harnstoffderivate, ihre Herstellung und ihre Verwendung als Lichtschutzmittel für Polymere

Die Erfindung betrifft neue Harnstoffderivate, deren Herstellung sowie ihre Verwendung zum Stabilisieren von synthetischen Polymeren gegen den zerstörenden Einfluss von Licht.

Die neuen Harnstoffderivate leiten sich vom 7,7,9,9-Tetramethyl-1-oxa-4-oxo-3,8-diaza-spiro-(4,5)-decan ab und besitzen die Struktur gemäss der Formel (I)

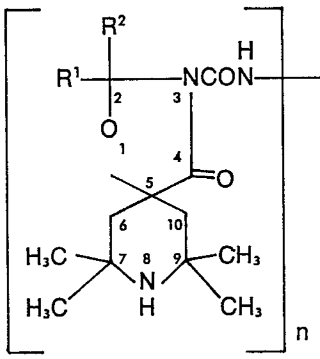

(I)

In dieser Formel bedeuten:

$R^1$ Wasserstoff, einen geradkettigen oder verzweigten Alkylrest mit 1 bis 10, vorzugsweise 1 bis 6 C-Atomen oder einen Aralkylrest mit 7 bis 10 C-Atomen, wobei die aliphatische Kette 1 bis 4 C-Atome besitzt,

$R^2$ ist eine Alkylgruppe mit 1 bis 17 und insbesondere 1 bis 11 C-Atomen, ein gegebenenfalls durch ein Halogenatom — bevorzugt Chlor — oder einen Alkylrest mit 1 bis 4 C-Atomen substituierter Phenyl- oder Naphthylrest oder ein Aralkylrest mit 7 bis 10 C-Atomen, wobei 1 bis 4 C-Atome zur aliphatischen Kette gehören,

$R^1$ und $R^2$ können auch zusammen mit dem sie bindenden Kohlenstoffatom einen Cycloalkanring mit 5 bis 12 und insbesondere 5 bis 7 C-Atomen bilden, welcher durch 1 oder 2 $C_1$ bis $C_4$-Alkylreste substituiert sein kann.

$R^3$ hat bei n = 1 die Bedeutung einer Alkylgruppe mit 1 bis 20, vorzugsweise 4 bis 18 C-Atomen, eines gegebenenfalls durch einen $C_1$- bis $C_4$-Alkylrest substituierten Cycloalkylrestes mit 5 bis 12, vorzugsweise 5 bis 7 und insbesondere 6 C-Atomen, eines gegebenenfalls durch ein Chloratom oder einen Alkylrest mit 1 bis 18, vorzugsweise 1 bis 4 C-Atomen substituierten Phenylrestes oder eines Phenylalkylrestes mit 7 bis 10 C-Atomen, während

$R^3$ bei n = 2 für eine geradkettige oder verzweigte Alkylengruppe mit 2 bis 12 insbesondere 2 bis 6 C-Atomen, oder für eine gegebenenfalls durch $C_1$- bis $C_4$-Alkyl substituierte Phenylengruppe oder für einen Diphenylenalkanrest mit 13 bis 18 C-Atomen steht.

Da das Ringstickstoffatom in Stellung 8 basische Eigenschaften aufweist, können die Verbindungen in diesen Fällen auch in Form von Salzen mit anorganischen oder organischen Säuren vorliegen.

Beispiele für $R^1$ sind Wasserstoff, Methyl, Äthyl, Propyl, Butyl, Pentyl, Hexyl, Heptyl, Benzyl, für $R^2$ Methyl, Äthyl, Propyl, Isopropyl, Butyl, Isobutyl, Pentyl, Isopentyl, Hexyl, Isohexyl, Heptyl, Isoheptyl, Octyl, Isooctyl, Nonyl, Isononyl, Decyl, Isodecyl, Undecyl Dodecyl, Tridecyl, Benzyl, Phenyl, Chlorphenyl, Phenyläthyl.

Beispiele für solche Fälle, in denen $R^1$ und $R^2$ zusammen mit dem Ring-C-Atom 2, an das sie gebunden sind, einen Cycloalkylring bilden, sind Cyclopentyl, Cyclohexyl, Methylcyclohexyl, Cycloheptyl und Cyclododecyl.

Beispiele für Reste $R^3$ bei Verbindungen mit n = 1 sind die Reste von Monoisocyanaten, z.B. Methyl, Äthyl, Propyl, Butyl, Isobutyl, Octadecyl, Cyclohexyl, Phenyl, 3-Chlorphenyl, 4-Chlorphenyl, 3-Methylphenyl, 4-Methylphenyl, 1-Naphthyl.

Bei n = 2 ist $R^3$ der Rest eines Diisocyanates, z.B. Äthylen, Hexamethylen, Diphenylenmethan, 4-Methyl-m-Phenylen.

Beispiele für mögliche Salze der Verbindungen der Formel (I) sind Salze mit nicht oxidierenden anorganischen Säuren wie Phosphate, Phosphite, Chloride, Sulfate und Salze mit organischen Mono- und Polycarbonsäuren wie Acetate, Laurate, Stearate, Succinate, Sebacate, Maleate, Citrate, Tartrate, Oxalate, Benzoate, Sulfonate, Phosphonate usw. Sie werden z.B. hergestellt, indem man äquivalente Mengen Säure und Base, jeweils in einem Lösungsmittel gelöst, miteinander vereinigt und das Lösungsmittel abdampft.

Von den neuen Harnstoffderivaten seien folgende beispielhaft aufgeführt:

2,2,7,7,9,9-Hexamethyl-3-propyl-carbamoyl-1-oxa-4-oxo-3,8-diaza-spiro-(4,5)-decan;
2,2,7,7,9,9-Hexamethyl-3-butyl-carbamoyl-1-oxa-4-oxo-3,8-diaza-spiro-(4,5)-decan;
2,2,7,7,9,9-Hexamethyl-3-tert.-butyl-carbamoyl-1-oxa-4-oxo-3,8-diaza-spiro-(4,5)-decan;
2,2,7,7,9,9-Hexamethyl-3-octadecyl-carbamoyl-1-oxa-4-oxo-3,8-diaza-spiro-(4,5)-decan;
2,2,7,7,9,9-Hexamethyl-3-cyclohexyl-carbamoyl-1-oxa-4-oxo-3,8-diaza-spiro-(4,5)-decan;
2,2,7,7,9,9-Hexamethyl-3-phenyl-carbamoyl-1-oxa-4-oxo-3,8-diaza-spiro-(4,5)-decan;
2,2,7,7,9,9-Hexamethyl-3-m-chlorphenyl-carbamoyl-1-oxa-4-oxo-3,8-diaza-spiro-(4,5)-decan;
2,2,7,7,9,9-Hexamethyl-3-p-chlorphenyl-carbamoyl-1-oxa-4-oxo-3,8-diaza-spiro-(4,5)-decan;
2,2,7,7,9,9-Hexamethyl-3-α-naphthyl-carbamoyl-1-oxa-4-oxo-3,8-diaza-spiro-(4,5)-decan;
2-Butyl-7,7,9,9-tetramethyl-3-octadecyl-carbamoyl-1-oxa-4-oxo-3,8-diaza-spiro-(4,5)-decan;
2-iso-Pentyl-7,7,9,9-tetramethyl-3-cyclohexyl-carbamoyl-1-oxa-4-oxo-3,8-diaza-spiro-(4,5)-decan;
2-iso-Nonyl-7,7,9,9-tetramethyl-3-butyl-carbamoyl-1-oxa-4-oxo-3,8-diaza-spiro-(4,5)-decan;
2-Undecyl-7,7,9,9-tetramethyl-3-octadecyl-carbamoyl-1-oxa-4-oxo-3,8-diaza-spiro-(4,5)-decan;

2-Phenyl-7,7,9,9-tetramethyl-3-phenyl-carbamoyl-1-oxa-4-oxo-3,8-diaza-spiro-(4,5)-decan;
2-Phenyl-7,7,9,9-tetramethyl-3-octadecyl-carbamoyl-1-oxa-4-oxo-3,8-diaza-spiro-(4,5)-decan;
2-Propyl-2,7,7,9,9-pentamethyl-3-propyl-carbamoyl-1-oxa-4-oxo-3,8-diaza-spiro-(4,5)-decan;
2-iso-Butyl-2,7,7,9,9-pentamethyl-3-cyclohexyl-carbamoyl-1-oxa-4-oxo-3,8-diaza-spiro-(4,5)-decan;
2-iso-Octyl-7,7,9,9-tetramethyl-3-cyclohexyl-carbamoyl-1-oxa-4-oxo-3,8-diaza-spiro-(4,5)-decan;
2-Nonyl-2,7,7,9,9-pentamethyl-3-octadecyl-carbamoyl-1-oxa-4-oxo-3,8-diaza-spiro-(4,5)-decan;
2,2-Diäthyl-7,7,9,9-tetramethyl-3-butyl-carbamoyl-1-oxa-4-oxo-3,8-diaza-spiro-(4,5)-decan;
2,2-Diäthyl-7,7,9,9-tetramethyl-3-cyclohexyl-carbamoyl-1-oxa-4-oxo-3,8-diaza-spiro-(4,5)-decan;
2,2-Diäthyl-7,7,9,9-tetramethyl-3-octadecyl-carbamoyl-1-oxa-4-oxo-3,8-diaza-spiro-(4,5)-decan;
2,2-Diäthyl-7,7,9,9-tetramethyl-3-phenyl-carbamoyl-1-oxa-4-oxo-3,8-diaza-spiro-(4,5)-decan;
2,2-Dipropyl-7,7,9,9-tetramethyl-3-butyl-carbamoyl-1-oxa-4-oxo-3,8-diaza-spiro-(4,5)-decan;
2,2-Dibutyl-7,7,9,9-tetramethyl-3-octadecyl-carbamoyl-1-oxa-4-oxo-3,8-diaza-spiro-(4,5)-decan;
2-Äthyl-2-pentyl-7,7,9,9-tetramethyl-3-cyclohexyl-carbamoyl-1-oxa-4-oxo-3,8-diaza-spiro-(4,5)-decan;
2,2-Dibenzyl-7,7,9,9-tetramethyl-3-phenyl-carbamoyl-1-oxa-4-oxo-3,8-diaza-spiro-(4,5)-decan;
2,2,4,4-Tetramethyl-4-butyl-carbamoyl-7-oxa-3,14-diaza-15-oxo-dispiro-(5,1,5,2)-pentadecan;
2,2,4,4-Tetramethyl-14-cyclohexyl-carbamoyl-7-oxa-3,14-diaza-15-oxo-dispiro-(5,1,5,2)-pentadecan;
2,2,4,4-Tetramethyl-14-phenyl-carbamoyl-7-oxa-3,14-diaza-15-oxo-dispiro-(5,1,5,2)-pentadecan;
n-Hexan-1',6'-bis-[3-carbamoyl-2,2,7,7,9,9-hexamethyl-1-oxa-4-oxo-3,8-diaza-spiro-(4,5)-decan];
Toluylen-2',4'-bis-[3-carbamoyl-2,2,7,7,9,9-hexamethyl-1-oxa-4-oxo-3,8-diaza-spiro-(4,5)-decan];
n-Hexan-1',6'-bis-[14-carbamoyl-2,2,4,4-tetramethyl-7-oxa-3,14-diaza-15-oxo-dispiro-(5,1,5,2)-pentadecan];
Toluylen-2',4'-bis-[14-carbamoyl-2,2,4,4-tetramethyl-7-oxa-3,14-diaza-15-oxo-dispiro-(5,1,5,2)-pentadecyl];
n-Hexan-1',6'-bis-[20-carbamoyl-2,2,4,4-tetramethyl-7-oxa-3,20-diaza-21-oxo-dispiro-(5,1,11,2)-heneicosan];
Toluylen-2',4'-bis-[20-carbamoyl-2,2,4,4-tetramethyl-7-oxa-3,20-diaza-21-oxo-dispiro-(5,1,11,2)-heneicosan];
n-Hexan-1',6'-bis-[3-carbamoyl-2,2-diäthyl-7,7,9,9-tetramethyl-1-oxa-4-oxo-3,8-diaza-spiro-(4,5)-decan];
Toluylen-2',4'-bis-[3-carbamoyl-2,2-diäthyl-7,7,9,9-tetramethyl-1-oxa-4-oxo-3,8-diaza-spiro-(4,5)-decan];
n-Hexan-1',6'-bis-[3-carbamoyl-2-iso-octyl-7,7,9,9-tetramethyl-1-oxa-4-oxo-3,8-diaza-spiro-(4,5)-decan];
Toluylen-2',4'-bis-[3-carbamoyl-2,2-diäthyl-7,7,9,9-tetramethyl-1-oxa-4-oxo-3,8-diaza-spiro-(4,5)-decan];
n-Hexan-1',6'-bis-[3-carbamoyl-2-iso-octyl-7,7,9,9-tetramethyl-1-oxa-4-oxo-3,8-diaza-spiro-(4,5)-decan].

Die abweichende Nomenklatur einiger der aufgezählten Verbindungen ergibt sich aus den IUPAC-Regeln (vgl. Hellwinkel, «Die systematische Nomenklatur der organischen Chemie»; Springer-Verlag, Heidelberg).

Die neuen Verbindungen der allgemeinen Formel (I) werden aus substituierten Piperidinen der allgemeinen Formel (III), die nach den Angaben der DE-OS 2 606 026 zugänglich sind, durch Umsetzen mit Isocyanaten bzw. Diisocyanaten (II) erhalten. Den Reaktionsablauf gibt die nachfolgende Gleichung wieder:

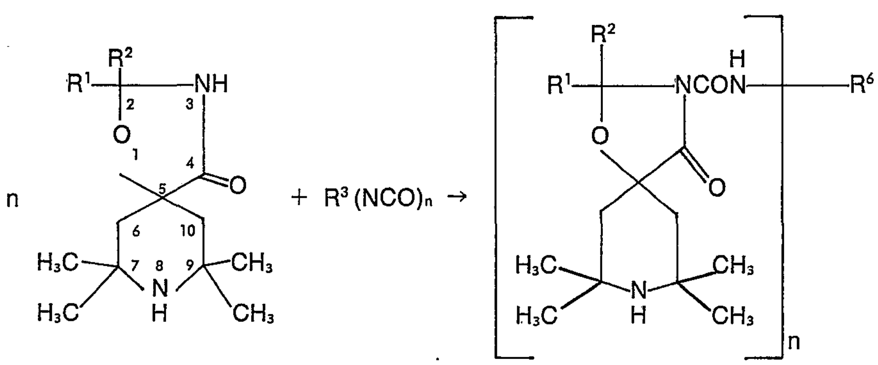

(III) (II) (I)

Die Reste $R^1$, $R^2$ und $R^3$ haben die früher angegebene Bedeutung. n ist 1 oder 2 entsprechend der Funktionalität des Isocyanats. Besonders überraschend ist, dass die Isocyanate nicht mit dem Aminstickstoff in der Stellung 8 reagieren.

Bei der Umsetzung geht man in der Weise vor, dass man das Piperidinderivat in einem inerten organischen Lösungsmittel vorlegt, eine katalytische Menge einer starken Base, wie z.B. 1,4-Diazabicyclooctan zusetzt, die äquivalente Menge des Isocyanats, gegebenenfalls in demselben Lösungsmittel gelöst, zutropft und bei 20 bis 180, vorzugsweise 20 bis 140 und insbesondere 50 bis 120°C reagieren lässt. Die Reaktionszeit beträgt 1 bis 30, vorzugsweise 4 bis 20 Stunden.

Geeignete inerte organische Lösungsmittel

sind z.B. Heptan, aliphatische Kohlenwasserstoffschnitte mit Siedebereichen bis 180°C, Toluol, Xylol, Diäthyläther, Dioxan, Halogenkohlenwasserstoffe wie Chloroform, Tetrachlorkohlenstoff und Chlorbenzol. Bevorzugt ist Toluol.

Schwer lösliche Reaktionsprodukte werden durch Filtration abgetrennt, gelöst bleibende reinigt man nach dem Abdampfen des Lösungsmittels durch Umkristallisation.

Die neuen Harnstoffderivate eignen sich hervorragend zum Stabilisieren von synthetischen Polymeren gegen die zersetzenden Einwirkungen von Licht, wobei unter «synthetischen Polymeren» in diesem Zusammenhang verstanden werden: halogenfreie Homo- und Copolymere, im einzelnen Homopolymerisate von Olefinen, Dienen und Styrol, wie z.B. Polyäthylen niedriger und hoher Dichte, Polypropylen, Polystyrol, Polybutadien und Polyisopren, Copolymere von Olefinen, Dienen und Styrol miteinander oder mit anderen olefinisch ungesättigten Monomeren, wie Äthylen-Propylen-Copolymere, Äthylen-Buten-Copolymere, Styrol-Butadien-Copolymere, Äthylen-Vinylacetat-Copolymere und Acrylnitril-Butadien-Styrol-Copolymere.

Des weiteren sollen auch Polyurethane, Polyacetale, Polyester, Polyamide, Polyacrylate und Epoxydharze eingeschlossen sein. Bevorzugt sind Poly-$\alpha$-Olefine wie Polyäthylene und insbesondere Polypropylene.

Es war überraschend und nicht vorhersehbar, dass die erfindungsgemässen Produkte bekannten, in den DE-A 2 500 313, 1 770 689 und 1 769 646 beschriebenen Verbindungen mit ähnlichen Strukturmerkmalen, d.h. Harnstoffen bzw. Spirodecanen mit ähnlichem Grundgerüst in ihrer UV-stabilisierenden Wirksamkeit merklich überlegen sind, es war vielmehr anzunehmen, dass aufgrund der an sich unwesentlich erscheinenden Veränderungen hinsichtlich der Struktur die Wirksamkeiten in derselben Grössenordnung liegen oder sogar schlechter sein sollten, weil bei den neuen Verbindungen wegen ihrer Polarität eine weniger gute Verträglichkeit, besonders mit unpolaren Polymeren, wie z.B. Polyolefinen, zu erwarten gewesen wäre. Desgleichen war aus diesem Grunde nicht zu erwarten, dass die neuen Produkte auch den Harnstoffderivaten der in der DE-A 2 606 026 beschriebenen Substanzen überlegen sein würden.

Die neuen stabilisierenden Verbindungen werden nach allgemein üblichen Methoden in die Polymermassen eingearbeitet. Alternativ kann man auch eine Lösung, Suspension oder Emulsion des Stabilisators mit dem Polymeren direkt oder mit einer Lösung, Suspension oder Emulsion derselben vermischen und das Lösungsmittel anschliessend entfernen.

Die erfindungsgemässen Stabilisatoren sind für sich allein oder im Gemisch mit einem oder mehreren der bei der Kunststoffverarbeitung üblichen Stabilisatoren, wie z.B. Antioxidantien auf Phenol- und Sulfidbasis, UV-Absorbern und Lichtschutzmitteln, Phosphitstabilisatoren, Metallverbindungen, Epoxydstabilisatoren u. mehrwertigen Alkoholen können ferner Flammschutzmittel und Pigmente, Farbstoffe, Antistatika und Füllstoffe, wie z.B. Glasfasern, anwesend sein.

Beispiele für geeignete Antioxidantien sind solche vom Typ der sterisch gehinderten Phenole wie 2,6-Di-tert.-butyl-p-kresol, 2,6-Dioctadecyl-p-kresol, 4,4'-Butyliden-bis-(2,6-di-tert.-butyl-phenol), 4,4'-Thio-bis-(2-tert.-butyl-5-methylphenol), phenolische Triazinverbindungen, Thiodipropionsäureester von Fettalkoholen, Dioctadecylsulfid und -disulfid.

Zu den UV-Absorbern und Lichtschutzmitteln gehören z.B. 2-(2'-Hydroxyphenyl)-benztriazole wie 2-(2'-Hydroxy-5'-methylphenyl)-benztriazol, 2-Hydroxybenzophenone wie 2-Hydroxy-4-octoxy-benzophenon, Stabilisatoren aus der Gruppe der Salizylate wie Octylphenylsalizylat, Nikkelchelate, Oxalsäurediamide und sterisch gehinderte Piperidinverbindungen.

Als Phosphitstabilisatoren sind Trisnonylphenylphosphit, Trislaurylphosphit oder auch Ester des Pentaerythritphosphits zu nennen.

Unter als Stabilisatoren bekannten Metallverbindungen werden verstanden: Calcium-, Barium-, Strontium-, Zink-, Cadmium-, Magnesium-, Aluminium- und Bleiseifen aliphatischer Carbonsäuren oder Oxycarbonsäuren mit etwa 12 bis 32 C-Atomen, Salze der genannten Metalle mit aromatischen Carbonsäuren wie Benzoate oder Salizylate sowie (Alkyl)-Phenolate dieser Metalle, ferner Organozinnverbindungen, wie z.B. Dialkylzinnthioglykolate und Carboxylate.

Bekannte Epoxydstabilisatoren sind z.B. epoxidierte höhere Fettsäuren wie epoxidiertes Sojabohnenöl, Tallöl, Leinöl oder epoxidiertes Butyloleat sowie Epoxide langkettiger Olefine.

Mehrwertige Alkohole können beispielsweise Pentaerythrit, Trimethylolpropan, Sorbit oder Mannit sein, d.h. bevorzugt Alkohole mit 5 oder 6 C-Atomen und 3 bis 6 OH-Gruppen.

Eine wirksame Stabilisatorkombination für Poly-$\alpha$-Olefine, wie z.B. Hoch-, Mittel- und Niederdruckpolymerisate von $C_2$- bis $C_4$-$\alpha$-Olefinen, insbesondere Polyäthylen und Polypropylen oder von Copolymerisaten derartiger $\alpha$-Olefine besteht, bezogen auf 100 Gewichtsteile Polymer, beispielsweise aus 0,01 bis 5 Gewichtsteilen einer der erfindungsgemäss zu verwendenden Verbindungen, 0,05 bis 5 Gewichtsteilen eines phenolischen Stabilisators, gegebenenfalls 0,01 bis 5 Gewichtsteilen eines schwefelhaltigen Costabilisators sowie gegebenenfalls 0,01 bis 3 Gewichtsteilen einer basischen oder neutralen Metallseife, wie z.B. Calciumstearat oder Zinkstearat sowie gegebenenfalls 0,1 bis 5 Gewichtsteilen eines Phosphits und gegebenenfalls 0,01 bis 5 Gewichtsteilen eines bekannten UV-Stabilisators aus der Gruppe der Alkoxyhydroxybenzophenone, Hydroxyphenylbenztriazole, Benzylidenmalonsäuremononitrilester oder der sog. Quencher, wie z.B. Nickelchelate.

Die folgenden Beispiele dienen der näheren Erläuterung der Erfindung. Die Strukturen der Verbindungen wurden kernresonanzspektroskopisch ermittelt, die Zusammensetzung wurde elementaranalytisch bestimmt.

Beispiel 1
2,2,7,7,9,9-Hexamethyl-3-propyl-carbamoyl-1--oxa-4-oxo-3,8-diaza-spiro-(4,5)-decan

12,0 g (0,05 Mol) 2,2,7,7,9,9-Hexamethyl-1-oxa--4-oxo-3,8-diaza-spiro-(4,5)-decan, 4,25 g (0,05 Mol) Propylisocyanat, 0,1 g 1,4-Diazabicyclooctan und 100 ml absolutes Toluol werden unter Rühren 15 Stunden rückfliessend gekocht. Anschliessend wird das Toluol abdestilliert und der Rückstand aus Heptan umkristallisiert. Fp. 119°C.

Stearat Fp. 87°C Succinat Fp. 169°C
p-tert.-Butylbenzoat Fp. 217°C.

Beispiele 2 bis 22

Entsprechend den Angaben des Beispiels 1 wurden hergestellt:

| Beispiel Nr. | Ausgangsmaterial Spiroverbindung | Isocyanat | Verfahrensprodukt Carbamoylverbindung | Fp.°C |
|---|---|---|---|---|
| 2 | 2,2,7,7,9,9-Hexamethyl-1-oxa-4--oxo-3,8-diaza-spiro-(4,5)-decan | $C_4H_9NCO$ | 2,2,7,7,9,9-Hexamethyl-3-butyl--carbamoyl-1-oxa-4-oxo-3,8-diaza--spiro-(4,5)-decan | 90-93 |
| 3 | 2,2,7,7,9,9-Hexamethyl-1-oxa-4--oxo-3,8-diaza-spiro-(4,5)-decan | t.-$C_4H_9NCO$ | 2,2,7,7,9,9-Hexamethyl-3-tert.-butyl--carbamoyl-1-oxa-4-oxo-3,8-diaza--spiro-(4,5)-decan | 95 |
| 4 | 2,2,7,7,9,9-Hexamethyl-1-oxa-4--oxo-3,8-diaza-spiro-(4,5)-decan | $C_{18}H_{37}NCO$ | 2,2,7,7,9,9-Hexamethyl-3-octadecyl--carbamoyl-1-oxa-4-oxo-3,8-diaza--spiro-(4,5)-decan | 51-53 |
| 5 | 2,2,7,7,9,9-Hexamethyl-1-oxa-4--oxo-3,8-diaza-spiro-(4,5)-decan | $C_6H_{11}NCO$ | 2,2,7,7,9,9-Hexamethyl-3-cyclohexyl--carbamoyl-1-oxa-4-oxo-3,8-diaza--spiro-(4,5)-decan | 123-125 |
| 6 | 2,2,7,7,9,9-Hexamethyl-1-oxa-4--oxo-3,8-diaza-spiro-(4,5)-decan | $C_6H_5NCO$ | 2,2,7,7,9,9-Hexamethyl-3-phenyl--carbamoyl-1-oxa-4-oxo-3,8-diaza--spiro-(4,5)-decan | 144-147 |
| 7 | 2,2,7,7,9,9-Hexamethyl-1-oxa-4--oxo-3,8-diaza-spiro-(4,5)-decan | $(CH_2)_6(NCO)_2$ | n-Hexan-1',6'-bis-[3-carbamoyl--2,2,7,7,9,9-hexamethyl-1-oxa-4-oxo--3,8-diaza-spiro-(4,5)-decan] | 191-194 |
| 8 | 2,2,7,7,9,9-Hexamethyl-1-oxa-4--oxo-3,8-diaza-spiro-(4,5)-decan | Toluylen-2,4--diisocyanat | Toluylen-2',4'-bis-[3-carbamoyl--2,2,7,7,9,9-hexamethyl-1-oxa-4-oxo--3,8-diaza-spiro-(4,5)-decan] | 186 |
| 9 | 2,2-Diäthyl-7,7,9,9-tetramethyl-1--oxa-4-oxo-3,8-diaza-spiro-(4,5)--decan | $C_4H_9NCO$ | 2,2-Diäthyl-7,7,9,9-tetramethyl-3--butyl-carbamoyl-1-oxa-4-oxo-3,8--diaza-spiro-(4,5)-decan | 105-108 |
| 10 | 2,2-Diäthyl-7,7,9,9-tetramethyl-1--oxa-4-oxo-3,8-diaza-spiro-(4,5)--decan | $C_6H_{11}NCO$ | 2,2-Diäthyl-7,7,9,9-tetramethyl-3--cyclohexyl-carbamoyl-1-oxa-4-oxo--3,8-diaza-spiro-(4,5)-decan | 72 |
| 11 | 2,2-Diäthyl-7,7,9,9-tetramethyl-1--oxa-4-oxo-3,8-diaza-spiro-(4,5)--decan | $C_6H_5NCO$ | 2,2-Diäthyl-7,7,9,9-tetramethyl-3--phenyl-carbamoyl-1-oxa-4-oxo--3,8-diaza-spiro-(4,5)-decan | 110-112 |
| 12 | 2,2-Diäthyl-7,7,9,9-tetramethyl-1--oxa-4-oxo-3,8-diaza-spiro-(4,5)--decan | $(CH_2)_6(NCO)_2$ | n-Hexan-1',6'-bis[3-carbamoyl-2,2--diäthyl-7,7,9,9-tetramethyl-1-oxa-4--oxo-3,8-diaza-spiro-(4,5)-decan] | 130-131 |
| 13 | 2,2-Diäthyl-7,7,9,9-tetramethyl-1--oxa-4-oxo-3,8-diaza-spiro-(4,5)--decan | Toluylen-2,4--diisocyanat | Toluylen-2',4'-bis-[3-carbamoyl-2,2--diäthyl-7,7,9,9-tetramethyl-1-oxa-4--oxo-3,8-diaza-spiro-(4,5)-decan] | 190 |
| 14 | 2-iso-Octyl-7,7,9,9-tetramethyl-1--oxa-4-oxo-3,8-diaza-spiro-(4,5)--decan | $C_6H_{11}NCO$ | 2-iso-Octyl-7,7,9,9-tetramethyl-3--cyclohexyl-carbamoyl-1-oxa-4-oxo--3,8-diaza-spiro-(4,5)-decan | 93-97 |

Fortsetzung Beispiele 2 bis 22

Entsprechend den Angaben des Beispiels 1 wurden hergestellt:

| Beispiel Nr. | Ausgangsmaterial Spiroverbindung | Isocyanat | Verfahrensprodukt Carbamoylverbindung | Fp.°C |
|---|---|---|---|---|
| 15 | 2-iso-Octyl-7,7,9,9-tetramethyl-1--oxa-4-oxo-3,8-diaza-spiro-(4,5)--decan | $(CH_2)_6(NCO)_2$ | n-Hexan-1',6'-bis-[3-carbamoyl-2--iso-octyl-7,7,9,9-tetramethyl-1-oxa--4-oxo-3,8-diaza-spiro-(4,5)-decan] | wachsartig |
| 16 | 2,2,4,4-Tetramethyl-7-oxa-3,14--diaza-15-oxo-dispiro-(5,1,5,2)--pentadecan | $C_4H_9NCO$ | 2,2,4,4-Tetramethyl-14-butyl-carbamoyl-7-oxa-3,14-diaza-15-oxo--dispiro-(5,1,5,2)-pentadecan | 103-106 |
| 17 | 2,2,4,4-Tetramethyl-7-oxa-3,14--diaza-15-oxo-dispiro-(5,1,5,2)--pentadecan | $C_6H_{11}NCO$ | 2,2,4,4-Tetramethyl-14-cyclohexyl--carbamoyl-7-oxa-3,14-diaza-15--oxo-dispiro-(5,1,5,2)-pentadecan | 153 |
| 18 | 2,2,4,4-Tetramethyl-7-oxa-3,14--diaza-15-oxo-dispiro-(5,1,5,2)--pentadecan | $C_6H_5NCO$ | 2,2,4,4-Tetramethyl-14-phenyl-carbamoyl-7-oxa-3,14-diaza-15-oxo--dispiro-(5,1,5,2)-pentadecan | 156-158 |
| 19 | 2,2,4,4-Tetramethyl-7-oxa-3,14--diaza-15-oxo-dispiro-(5,1,5,2)--pentadecan | $(CH_2)_6(NCO)_2$ | n-Hexan-1',6'-bis-[14-carbamoyl--2,2,4,4-tetramethyl-7-oxa-3,14-diaza--15-oxo-dispiro-(5,1,5,2)-pentadecan] | 154-156 |
| 20 | 2,2,4,4-Tetramethyl-7-oxa-3,14--diaza-15-oxo-dispiro-(5,1,5,2)--pentadecan | Toluylen-2,4--diisocyanat | Toluylen-2',4'-bis-[14-carbamoyl--2,2,4,4-tetramethyl-7-oxa-3,14-diaza--15-oxo-dispiro-(5,1,5,2)-pentadecan] | 158 |
| 21 | 2,2,4,4-Tetramethyl-7-oxa-3,20--diaza-21-oxo-dispiro-(5,1,11,2)--heneicosan | $(CH_2)_6(NCO)_2$ | n-Hexan-1',6'-bis-[20-carbamoyl--2,2,4,4-tetramethyl-7-oxa-3,20-diaza--21-oxo-dispiro-(5,1,11,2)-heneicosan] | 135 |
| 22 | 2,2,4,4-Tetramethyl-7-oxa-3,20--diaza-21-oxo-dispiro-(5,1,11,2)--heneicosan | Toluylen-2,4--diisocyanat | Toluylen-2',4'-bis-[20-carbamoyl--2,2,4,4-tetramethyl-7-oxa-3,20-diaza--21-oxo-dispiro-(5,1,11,2)-heneicosan] | 140 |

Beispiel 23

Dieses Beispiel zeigt die lichtstabilisierende Wirkung der erfindungsgemässen Verbindungen beim Einsatz in einem Poly-α-Olefin.

100 Gewichtsteile Polypropylen mit einem Schmelzindex $i_5$ von ca. 6 g/10 Min. (bestimmt nach ASTM D 1238-62 T) und einer Dichte von 0,96 wurden mit

0,1 Gew.-Teilen Pentaerythrityl-tetrakis-3-(3,5--di-tert.-butyl-4-hydroxyphenyl)--propionat,
0,2 Gew.-Teilen Calciumstearat und
0,1 Gew.-Teilen des zu prüfenden erfindungsgemässen Stabilisators vermischt.

Um eine möglichst gleichmässige Verteilung auf dem Polymerkorn zu erreichen, wurden die Stabilisatoren in einem Lösungsmittel gelöst, und die Lösung wurde unter Rühren in das Polypropylenpulver eingetropft, wobei durch gleichzeitige Bestrahlung mit einer IR-Lampe der grösste Teil des Lösemittels wieder abdampfte. Nach ca. 20 Min. wurde das Calciumstearat hinzugegeben und noch weitere 10 Min. gemischt. Lösemittelreste wurden durch Trocknen bei 50°C/120 Min. im Trockenschrank entfernt.

Daraus wurden auf einer Windsor-Spritzgussmaschine der Type SP 50 bei 250°C zu 60 × 60 × 1 mm-Platten verspritzt. Aus diesen Platten wurden Prüfkörper nach DIN 53 455, Form 3, verkleinert im Massstab 1 : 3, ausgestanzt. Die als Vergleichsmuster benötigten Prüfkörper wurden analog, jedoch unter Fortlassen des zu testenden Stabilisators (Versuch g) bzw. unter Verwendung bekannter Lichtstabilisatoren (Versuche e und f), hergestellt.

Die Prüfung der Lichtbeständigkeit wurde in einer Xenotest-450-Apparatur der Firma Original Hanau Quarzlampen GmbH mit der Filterkombination 6 IR + 1 UV gemäss DIN 53 387 «Kurzprüfung der Wetterbeständigkeit» geprüft. Während der Belichtungszeit beträgt die Schwarztafeltemperatur 43°C ± 1°C, die relative Luftfeuchtigkeit im Probenraum 70% ± 1%. Der Probenraum wurde alle zwei Stunden 5 Min. lang mit Frischluft gespült. Die Reissdehnung wurde auf einer Zugprüfmaschine der Firma Instron bei einer Abzugsgeschwindigkeit von 5 cm/Min. nach einer definierten Belichtungszeit ermittelt. Die Ergebnisse sind in der nachstehenden Tabelle zusammengestellt.

Der Stabilitätsfaktor ergibt sich aus dem Verhältnis der Bestrahlungszeit des stabilisierten zur Bestrahlungszeit des nicht stabilisierten Probekörpers, wobei jeweils so lange bestrahlt wurde, bis die Reissdehnung auf die Hälfte des Ausgangswertes gefallen ist.

| Versuch | Stabilisator nach Beispiel | Stabilitätsfaktor |
|---|---|---|
| a) | 5 | 4,5 |
| b) | 8 | 4,5 |
| c) | 19 | 5,0 |
| d) | 21 | 5,0 |
| e) | Benzophenonstabilisator [1)] | 2,5 |
| f) | Benzotriazolstabilisator [2)] | 2,5 |
| g) | Kontrolle (ohne Stabilisator) | 1 |

[1)] 2-Hydroxy-4-n-octyloxybenzophenon
[2)] 2-(2-Hydroxy-3',5'-di-tert.-butylphenyl-5-chlorbenzotriazol

**Patentansprüche**

1. Harnstoffderivate der allgemeinen Formel I

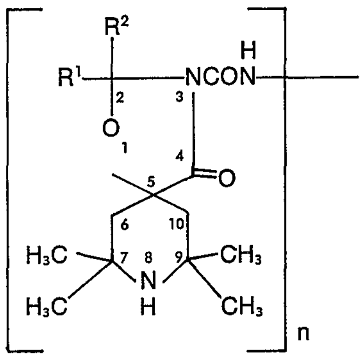

(I)

in der
$R^1$ für Wasserstoff oder einen geradkettigen oder verzweigten Alkylrest mit 1 bis 10 C-Atomen oder einen Aralkylrest mit 7 bis 10 C-Atomen steht,
$R^2$ eine Alkylgruppe mit 1 bis 17 C-Atomen ein gegebenenfalls durch ein Halogenatom oder einen Alkylrest mit 1 bis 4 C-Atomen substituierter Phenyl- oder Naphthylrest oder ein Aralkylrest mit 7 bis 10 C-Atomen ist, oder auch
$R^1$ und $R^2$ mit dem sie bindenden Kohlenstoffatom einen Cycloalkanring mit 5 bis 12 C-Atomen bilden, wobei dieser Cycloalkanring durch $C_1$- bis $C_4$-Alkylreste substituiert sein kann, und
n entsprechend der Wertigkeit von $R^3$ für 1 oder 2 steht, wobei
$R^3$ bei n = 1 die Bedeutung einer Alkylgruppe mit 1 bis 20 C-Atomen, eines gegebenenfalls durch einen $C_1$- bis $C_4$-Alkylrest substituierten Cycloalkylrestes mit 5 bis 12 C-Atomen, eines gegebenenfalls durch ein Chloratom oder einen Alkylrest mit 1 bis 18 C-Atomen substituierten Phenylrestes oder eines Phenylrestes mit 7 bis 10 C-Atomen hat, während
$R^3$ bei n = 2 eine geradkettige oder verzweigte Alkylengruppe mit 2 bis 12 C-Atomen, eine gegebenenfalls durch $C_1$- bis $C_4$-Alkyl substituierte Phenylengruppe oder ein Diphenylenalkanrest mit 13 bis 18 C-Atomen ist.

2. Verfahren zur Herstellung von Verbindungen nach Anspruch 1, dadurch gekennzeichnet, dass man in einem inerten organischen Lösungsmittel bei Temperaturen von 20 bis 180°C unter Zusatz katalytischer Mengen einer Base eine Verbindung der Formel (III)

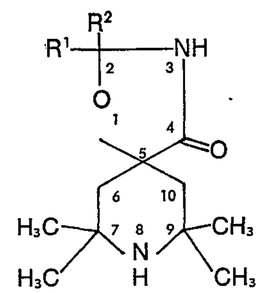

(III)

mit einem Isocyanat der Formel (II)

$$R^3(NCO)_n \qquad (II)$$

wobei die Reste $R^1$, $R^2$ und $R^3$ und der Index n die in Anspruch 1 angegebene Bedeutung haben, umsetzt.

3. Verwendung der Verbindungen nach Anspruch 1, gegebenenfalls in Form des Salzes mit einer anorganischen oder organischen Säure, zum Stabilisieren von synthetischen Polymeren.

4. Verwendung nach Anspruch 3, dadurch gekennzeichnet, dass das Polymere ein Polyolefin ist.

5. Verfahren zum Stabilisieren von synthetischen Polymeren gegen den schädigenden Einfluss von Licht, dadurch gekennzeichnet, dass man den Polymeren, gegebenenfalls neben bisher bekannten, stabilisierend wirkenden Stoffen, 0,01 bis 5 Gewichtsteile, bezogen auf Polymeres, einer Verbindung nach Anspruch 1 zusetzt, wobei diese Verbindung auch in Form eines Salzes mit einer anorganischen oder organischen Säure vorliegen kann.

**Claims**

1. Urea derivatives of the formula (I)

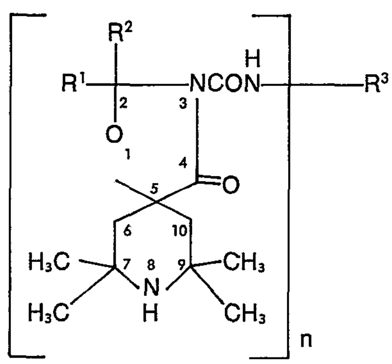

(I)

in which
$R^1$ denotes hydrogen, or a straight chain or branched alkyl group having from 1 to 10 carbon atoms, or aralkyl having from 7 to 10 carbon atoms;
$R^2$ denotes an alkyl group having from 1 to 17 carbon atoms, phenyl or naphthyl which may be substituted by a halogen atom, or alkyl having from 1 to 4 carbon atoms, or denotes aralkyl with 7 to 10 carbon atoms, or
$R^1$ and $R^2$ may also be together with the carbon atom by which they are linked a cycloalkane ring having from 5 to 12 carbon atoms which may be substituted by $C_1$ to $C_4$ alkyl radicals;
n is 1 or 2 depending on the valence of $R^3$,
$R^3$, with n being 1, denotes alkyl having from 1 to 20 carbon atoms, cycloalkyl having from 5 to 12 carbon atoms which may be substituted by a $C_1$-$C_4$-alkyl radical; phenyl which may be substituted by a chlorine atom or alkyl having from 1 to 18 carbon atoms; or phenylalkyl having from 7 to 10 carbon atoms, or
$R^3$, with n being 2, denotes a straight chain or branched alkylene group having from 2 to 12 carbon atoms, or phenylene which may be substituted by $C_1$-$C_4$-alkyl, or a diphenylene-alkane radical having from 13 to 18 carbon atoms.

2. Process for the manufacture of compounds as claimed in claim 1, which comprises reacting a compound of the formula (III)

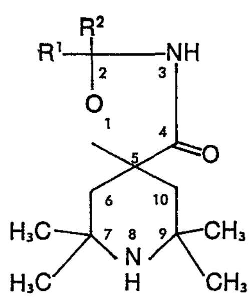

(III)

in an inert organic solvent, at a temperature of 20 to 180°C and with the addition of catalytic amount of a base, with an isocyanate of the formula (II)

$$R^3(NCO)_n \qquad (II)$$

in which formulae $R^1$, $R^2$, $R^3$ and n are as defined in claim 1.

3. Use of a compound as claimed in claim 1 or of a salt thereof with an inorganic or organic acid for stabilizing synthetic polymers.

4. Use as claimed in claim 3, wherein the polymer is a polyolefin.

5. Process for stabilizing synthetic polymers against the detrimental action of light, which comprises adding to the polymers, optionally in addition to hitherto known substances with stabilizing action, of from 0.01 to 5 weight parts, referred to the polymer, of a compound as claimed in claim 1, said compound being optionally present in the form of a salt with an inorganic or organic acid.

## Revendications

1. Dérivés de l'urée répondant à la formule générale (I)

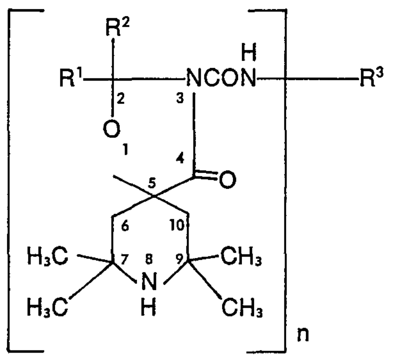

(I)

dans laquelle:
$R^1$ représente l'hydrogène ou un radical alkyle, linéaire ou ramifié, contenant de 1 à 10 atomes de carbone, ou un radical aralkyle contenant de 7 à 10 atomes de carbone.
$R^2$ représente un radical alkyle contenant de 1 à 17 atomes de carbone, un radical phényle ou naphtyle éventuellement porteur d'un atome d'halogène ou d'un radical alkyle en $C_1$-$C_4$, ou un radical aralkyle contenant de 7 à 10 atomes de carbone, ou encore,
$R^1$ et $R^2$ forment ensemble et avec l'atome de carbone qui les unit un noyau de cycloalcane contenant de 5 à 12 atomes de carbone, ce noyau de cycloalcane pouvant lui-même porter des radicaux alkyles en $C_1$-$C_4$,
n est égal à 1 ou à 2 suivant la valence de $R^3$, et
$R^3$, dans le cas ou n est égal à 1, représente un radical alkyle en $C_1$-$C_{20}$, un radical cycloalkyle en $C_5$-$C_{12}$ éventuellement porteur d'un radical alkyle en $C_1$-$C_4$, un radical phényle éventuellement porteur d'un atome de chlore ou d'un radical alkyle en $C_1$-$C_{18}$, ou un radical phénylalkyle en $C_7$-$C_{10}$, et, lorsque n est égal à 2, $R^3$ représente un radical alkylène, linéaire ou ramifié, contenant de 2 à 12 atomes de carbone, un radical phénylène éventuellement porteur d'un radical alkyle en $C_1$-$C_4$, ou un radical diphénylène-alcane contenant de 13 à 18 atomes de carbone.

2. Procédé de préparation de composés selon la revendication 1, procédé caractérisé en ce qu'on fait réagir, dans un solvant organique inerte, à des températures de 20 à 180°C, en présence de quantités catalytiques d'une base, un composé répondant à la formule (III)

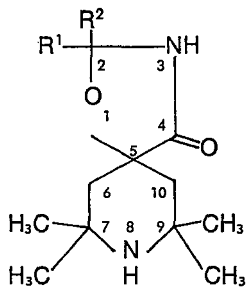

(III)

avec un isocyanate répondant à la formule (II)

$$R^3(NCO)_n \qquad (II)$$

formules dans lesquelles $R^1$, $R^2$, $R^3$ et n ont les significations données à la revendication 1.

3. Application des composés selon la revendication 1, éventuellement sous la forme de sels avec des acides minéraux ou organiques, pour la stabilisation de polymères synthétiques.

4. Application selon la revendication 3, caractérisé en ce que le polymère est une polyoléfine.

5. Procédé pour stabiliser des polymères synthétiques contre les effets destructeurs de la lumière, procédé caractérisé en ce qu'on ajoute aux polymères, éventuellement en plus de stabilisant connus, de 0,01 à 5% en poids, par rapport aux polymères, d'un composé selon la revendication 1, ce composé pouvant également être sous la forme d'un sel avec un acide minéral ou organique.